# EUROPEAN PATENT SPECIFICATION

(11) **EP 1 483 392 B1**
(45) Date of publication and mention of the grant of the patent: **01.12.2010**
(21) Application number: 03706581.0
(22) Date of filing: 27.02.2003
(51) Int. Cl.: C12P 13/04, C12P 13/08, C12N 1/21, C12N 15/11, C12N 15/57

(54) **PROCESS FOR THE PREPARATION OF L-AMINO ACIDS USING STRAINS OF THE FAMILY ENTEROBACTERIACEAE**
VERFAHREN ZUR HERSTELLUNG VON L-AMINOSÄUREN UNTER VERWENDUNG VON STÄMMEN AUS DER FAMILIE DER ENTEROBACTERIACEAE
PROCEDE DE PREPARATION DE L-AMINOACIDES AU MOYEN DE SOUCHES DE LA FAMILLE DES ENTEROBACTERIACEES

(30) Priority: 13.03.2002 DE 10210965; 21.03.2002 US 365838 P
(43) Date of publication of application: 08.12.2004
(73) Proprietor: Evonik Degussa GmbH, 45128 Essen (DE)
(72) Inventor: RIEPING, Mechthild, 33619 Bielefeld (DE); HERMANN, Thomas, 33739 Bielefeld (DE)
(86) International application number: PCT/EP2003/001998
(87) International publication number: WO 2003/076637

(56) References cited:
- WO-A-02/101063
- US-A- 4 278 765
- SUZUKI ET AL.: "Aminopeptidases A, B, and N and Dipeptidase D are the foru cysteinylglycinases of Escherichia coli K-12" J. BACTERIOL., vol. 183, no. 4, - 1 February 2001 (2001-02-01) pages 1489-1490, XP002243880 cited in the application
- MATHEW ET AL.: "Salmonella enterica Serovar Typhimurium peptidase B is a leucyl aminopeptidase with specificity with acidic amino acids" J. BACTERIOL., vol. 182, no. 12, - June 2000 (2000-06) pages 3383-3393, XP002243881
- SUZUKI H ET AL: "MAPPING, CLONING, AND DNA SEQUENCING OF PEPB WHICH ENCODES PEPTIDASE B OF ESCHERICHIA COLI K-12" JOURNAL OF FERMENTATION AND BIOENGINEERING, SOCIETY OF FERMENTATION TECHNOLOGY, JP, vol. 82, no. 4, 1996, pages 392-397, XP008016110 ISSN: 0922-338X cited in the application
- CHASSAGNOLE C ET AL: "An integrated study of threonine-pathway enzyme kinetics in Escherichia coli" BIOCHEMICAL JOURNAL, THE BIOCHEMICAL SOCIETY, LONDON, GB, vol. 356, no. 2, 1 June 2001 (2001-06-01), pages 415-423, XP002232684 ISSN: 0264-6021

## Description

The present invention relates to a fermentation process for the preparation of L-threonine, using strains of the family Enterobacteriaceae in which the pepB gene is enhanced..

### State of the art

L-Amino acids, especially L-threonine, are used in human medicine and in the pharmaceutical industry, in the food industry and very particularly in animal nutrition.

It is known to prepare L-amino acids by the fermentation of strains of Enterobacteriaceae, especially Escherichia coli (E. coli) and Serratia marcescens. Because of their great importance, attempts are constantly being made to improve the preparative processes. Improvements to the processes may relate to measures involving the fermentation technology, e.g.. stirring and oxygen supply, or the composition of the nutrient media, e.g. the sugar concentration during fermentation, or the work-up to the product form, e.g. by ion exchange chromatography, or the intrinsic productivity characteristics of the microorganism itself..

The productivity characteristics of these microorganisms are improved by using methods of mutagenesis, selection and mutant choice to give strains which are resistant to antimetabolites, e.g. the threonine analog α-amino-β-hydroxyvaleric acid (AHV), or auxotrophic for metabolites of regulatory significance, and produce L-amino acids, e.g. L-threonine.

Methods of recombinant DNA technology have also been used for some years to improve L-amino acid-producing strains of the family Enterobacteriaceae by amplifying individual amino acid biosynthesis genes and studying the effect on production.

Mathew et al describe in "Salmonella enterica Serovar Typhimurium peptidase B is a leucyl aminopeptidase with specifity with acidic amino acids" J. Bacteriol., Vol. 182, no. 12, - June 2000 (2000-06) pages 3383-3393, XP002243881, that peptidase B from Salmonella enterica (Enterobacteriaceae) hydrolyzes peptides with N-terminal Asp or Glu residues.

Suzuki H. et al disclose in "Mapping, cloning, and DNA Sequencing of pepB which encodes peptidase B of Escherichia Coli K-12" Journal of Fermentation and Bioengineering, Society of Fermentation Technology, JP, vol. 82, no. 4, 1996, pages 392-397, XP008016110 ISSN: 0922-338X, E. Coli SH 949 which has been transformed with the plasmid pNS42 comprising the pepB gene and which over-produced peptidase B.

It is described by Chassagnole C. et al. in "An integrated study of threonine-pathway enzyme kinetics in Escherichia coli" Biochemical Journal, The Biochemical Society, London, GB, Vol. 356, no. 2, 1 June 2001 (2001-06-01), pages 415-423, XP002232684 ISSN: 0264-6021, the Thr metabolism in E. coli and disclosed Asp as precursor of L-threonine.

WO 02/101063 (Degussa) discloses a fermentation process for the preparation of D-pantothenic acid using microorganisms of the Enterobacteriaceae family in which the pepB gene is enhanced.

### Object of the invention

The object which the inventors set themselves was to provide novel procedures for improving the preparation of L-threonine, by fermentation.

### Description of the invention

The invention provides a fermentation process for the preparation of L-threonine, using microorganisms of the family Enterobacteriaceae which, in particular, already produce L-threonine and in which the nucleotide sequence coding for the pepB gene product is enhanced.

The term "L-threonine mentioned hereafter is to be understood as meaning the amino acid, including its salt.

In this context the term "enhancement" describes the increase, in a microorganism, of the intracellular activity of one or more enzymes or proteins coded for by the appropriate DNA, for example by increasing the copy number of the gene or genes, using a strong promoter or a gene or allele coding for an appropriate enzyme or protein with a high activity, and optionally combining these measures.

Through the measures of enhancement, especially overexpression, the activity or concentration of the appropriate protein is generally increased at least by 10%, 25%, 50%, 75%, 100%, 150%, 200%, 300%, 400% or 500%, and at most by up to 1000% or 2000%, based on that of the wild-type protein or the activity or concentration of the protein in the starting microorganism.

The process is **characterised in that** the following steps are carried out:
a) fermentation of microorganisms of the family Enterobacteriaceae which produce the L-threonine, and in which the pepB gene, or nucleotide sequences coding for the pepB gene product, is (are) enhanced and, in particular, overexpressed,
b) enrichment of L-threonine in the medium or in the cells of the microorganisms, and
c) isolation of L-threonine, where all or part (≥ 0 to 100) of the biomass, optionally remain in the produced L-threonine.

The microorganisms provided by the present invention can produce L-amino acids from glucose, sucrose, lactose, fructose, maltose, molasses, optionally starch or optionally cellulose, or from glycerol and ethanol. Said microorganisms are representatives of the family Enterobacteriaceae selected from the genera Escherichia, Erwinia, Providencia and Serratia. The genera Escherichia and Serratia are preferred. The species Escherichia coli and Serratia marcescens may be mentioned in particular among the genera Escherichia and Serratia respectively. Examples of suitable strains, particularly L-threonine-producing strains, of the genus Escherichia, and especially of the species Escherichia coli, are:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132

Examples of suitable L-threonine-producing strains of the genus Serratia, and especially of the species Serratia marcescens, are:
Serratia marcescens HNr21
Serratia marcescens TLr156
Serratia marcescens T2000

L-Threonine-producing strains of the family Enterobacteriaceae preferably possess, inter alia, one or more genetic or phenotypic characteristics selected from the group comprising resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidine, resistance to rifampicin, resistance to valine analogs such as valine hydroxamate, resistance to purine analogs such as 6-dimethylaminopurine, need for L-methionine, optionally partial and compensatable need for L-isoleucine, need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally capability for sucrose utilization, enhancement of the threonine operon, enhancement of homoserine dehydrogenase I-aspartate kinase I, preferably of the feedback-resistant form, enhancement of homoserine kinase, enhancement of threonine synthase, enhancement of aspartate kinase, optionally of the feedback-resistant form, enhancement of aspartate semialdehyde dehydrogenase, enhancement of phosphoenolpyruvate carboxylase, optionally of the feedback-resistant form, enhancement of phosphoenolpyruvate synthase, enhancement of transhydrogenase, enhancement of the RhtB gene product, enhancement of the RhtC gene product, enhancement of the YfiK gene product, enhancement of a pyruvate carboxylase and attenuation of acetic acid formation.

It has been found that the production of L-threonine, by microorganisms of the family Enterobacteriaceae is improved after enhancement and, in particular, overexpression of the pepB gene.

The nucleotide sequences of the genes of Escherichia coli belong to the state of the art (cf. literature references below) and can also be taken from the genome sequence of Escherichia coli published by Blattner et al. (Science 277, 1453-1462 (1997)).

The pepB gene is described inter alia by the following data:

| | |
|---|---|
| Name: | aminopeptidase B, cysteinylglycinase |
| Reference: | Suzuki et al.; Journal of Fermentation and Bioengineering 82, 392-397 (1996) Suzuki et al.; Bioscience, Biotechnology and Biochemistry 65(7), 1549-1558 (2001) |
| Accession no.: | AE000339 |

The nucleic acid sequences can be taken from the data banks of the National Center for Biotechnology Information (NCBI) of the National Library of Medicine (Bethesda, MD, USA), the nucleotide sequence data bank of the European Molecular Biologies Laboratories (EMBL, Heidelberg, Germany, or Cambridge, UK) or the DNA Databank of Japan (DDBJ, Mishima, Japan).

The genes described in the literature references cited can be used according to the invention. It is also possible to use alleles of the genes which result from the degeneracy of the genetic code or from neutral sense mutations. The use of endogenous genes is preferred.

The term "endogenous genes" or "endogenous nucleotide sequences" is to be understood as meaning the genes or alleles, or nucleotide sequences, present in the population of a species.

Enhancement can be achieved for example by increasing the expression of the genes or enhancing the catalytic properties of the proteins. Both measures may optionally be combined.

The peptidase and cysteinylglycinase activity of aminopeptidase B, which is coded for by the polynucleotide pepB, can be determined by the method described by Suzuki et al.. (Journal of Bacteriology 183(4), 1489-1490 (2001)).

Overexpression can be achieved by increasing the copy number of the appropriate genes or mutating the promoter and regulatory region or the ribosome binding site located upstream from the structural gene. Expression cassettes incorporated upstream from the structural gene work in the same way. Inducible promoters additionally make it possible to increase expression in the course of L-threonine production by fermentation.. Measures for prolonging the life of the mRNA also improve expression. Furthermore, the enzyme activity is also enhanced by preventing the degradation of the enzyme protein. The genes or gene constructs can either be located in plasmids of variable copy number or be integrated and amplified in the chromosome. Alternatively, it is also possible to achieve overexpression of the genes in question by changing the composition of the media and the culture technique..

Those skilled in the art will find relevant instructions inter alia in Chang and Cohen (Journal of Bacteriology 134, 1141-1156 (1978)), Hartley and Gregori (Gene 13, 347-353 (1981)), Amann and Brosius (Gene 40, 183-190 (1985)), de Broer et al. (Proceedings of the National Academy of Sciences of the United States of America 80, 21-25 (1983)), LaVallie et al. (BIO/TECHNOLOGY 11, 187-193 (1993)), PCT/US97/13359, Llosa et al. (Plasmid 26, 222-224 (1991)), Quandt and Klipp (Gene 80, 161-169 (1989)), Hamilton et al. (Journal of Bacteriology 171, 4617-4622 (1989)), Jensen and Hammer (Biotechnology and Bioengineering 58, 191-195 (1998)) and well-known textbooks on genetics and molecular biology.

Plasmid vectors replicatable in Enterobacteriaceae, e.g. cloning vectors derived from pACYC184 (Bartolomé et al.; Gene 102, 75-78 (1991)), pTrc99A (Amann et al.; Gene 69, 301-315 (1988)) or pSC101 derivatives .(Vocke and Bastia; Proceedings of the National Academy of Sciences USA 80(21), 6557-6561 (1983)), can be used. In one process according to the invention, it is possible to use a strain transformed with a plasmid vector, said plasmid vector carrying at least one nucleotide sequence coding for the pepB gene.

Also, mutations which affect the expression of the appropriate genes can be transferred to different strains by sequence exchange (Hamilton et al.; Journal of Bacteriology 171, 4617-4622 (1989)), conjugation or transduction.

Furthermore., for the production of L-threonine, with strains of the family Enterobacteriaceae, it can be advantageous not only to enhance the pepB gene but also to enhance one or more enzymes of the known threonine biosynthetic pathway, or enzymes of the anaplerotic metabolism, or enzymes for the production of reduced nicotinamide adenine dinucleotide phosphate, or glycolytic enzymes, or PTS enzymes or enzymes of sulfur metabolism. The use of endogenous genes is generally preferred.

Thus, for example, one or more genes selected from the group comprising:
- the thrABC operon coding for aspartate kinase, homoserine dehydrogenase, homoserine kinase and threonine synthase (US-A-4,278,765),
- the pyc gene coding for pyruvate carboxylase (DE-A-19 831 609),
- the pps gene coding for phosphoenolpyruvate synthase (Molecular and General Genetics 231(2), 332-336 (1992)),
- the ppc gene coding for phosphoenolpyruvate carboxylase (Gene 31, 279-283 (1984)),
- the pntA and pntB genes coding for transhydrogenase (European Journal of Biochemistry 158, 647-653 (1986)),
- the rhtB gene for homoserine resistance (EP-A-0 994 190),
- the mqo gene coding for malate:quinone oxidoreductase (DE 100 348 33.5),
- the rhtC gene for threonine resistance (EP-A-1 013 765),
- the thrE gene of Corynebacterium glutamicum coding for threonine export protein (DE 100 264 94.8),
- the gdhA gene coding for glutamate dehydrogenase (Nucleic Acids Research 11, 5257-5266 (1983); Gene 23, 199-209 (1983)),
- the hns gene coding for DNA binding protein HLP-II (Molecular and General Genetics 212, 199-202 (1988)),
- the pgm gene coding for phosphoglucomutase (Journal of Bacteriology 176, 5847-5851 (1994)),
- the fba gene coding for fructose biphosphate aldolase (Biochemical Journal 257, 529-534 (1989)),
- the ptsH gene of the ptsHIcrr operon coding for phosphohistidine protein hexose phosphotransferase of the phosphotransferase system PTS (Journal of Biological Chemistry 262, 16241-16253 (1987)),
- the ptsI gene of the ptsHIcrr operon coding for enzyme I of the phosphotransferase system PTS (Journal of Biological Chemistry 262, 16241-16253 (1987)),
- the crr gene of the ptsHIcrr operon coding for the glucose-specific IIA component of the phosphotransferase system PTS (Journal of Biological Chemistry 262, 16241-16253 (1987)),
- the ptsG gene coding for the glucose-specific IIBC component (Journal of Biological Chemistry 261, 16398-16403 (1986)),
- the lrp gene coding for the regulator of the leucine regulon (Journal of Biological Chemistry 266, 10768-10774 (1991)),
- the csrA gene coding for the global regulator Csr (Journal of Bacteriology 175, 4744-4755 (1993)),
- the fadR gene coding for the regulator of the fad regulon (Nucleic Acids Research 16, 7995-8009 (1988)),
- the iclR gene coding for the regulator of the central intermediary metabolism (Journal of Bacteriology 172, 2642-2649 (1990)),
- the mopB gene coding for the 10 kd chaperone (Journal of Biological Chemistry 261, 12414-12419 (1986)), which is also known as groES,
- the ahpC gene of the ahpCF operon coding for the small subunit of alkyl hydroperoxide reductase (Proceedings of the National Academy of Sciences USA 92, 7617-7621 (1995)),
- the ahpF gene of the ahpCF operon coding for the large subunit of alkyl hydroperoxide reductase (Proceedings of the National Academy of Sciences USA 92, 7617-7621 (1995)),
- the cysK gene coding for cysteine synthase A (Journal of Bacteriology 170, 3150-3157 (1988)),
- the cysB gene coding for the regulator of the cys regulon (Journal of Biological Chemistry 262, 5999-6005 (1987)),
- the cysJ gene of the cysJIH operon coding for the flavoprotein of NADPH sulfite reductase (Journal of Biological Chemistry 264, 15796-15808 (1989), Journal of Biological Chemistry 264, 15726-15737 (1989)),
- the cysI gene of the cysJIH operon coding for the hemoprotein of NADPH sulfite reductase (Journal of Biological Chemistry 264, 15796-15808 (1989), Journal of Biological Chemistry 264, 15726-15737 (1989)),
- the cysH gene of the cysJIH operon coding for adenylyl sulfate reductase (Journal of Biological Chemistry 264, 15796-15808 (1989), Journal of Biological Chemistry 264, 15726-15737 (1989)),
- the phoB gene of the phoBR operon coding for the PhoB positive regulator of the pho regulon (Journal of Molecular Biology 190 (1), 37-44 (1986)),
- the phoR gene of the phoBR operon coding for the sensor protein of the pho regulon (Journal of Molecular Biology 192 (3), 549-556 (1986)),
- the phoE gene coding for protein E of the outer cell membrane (Journal of Molecular Biology 163 (4), 513-532 (1983)),
- the pykF gene coding for fructose-stimulated pyruvate kinase I (Journal of Bacteriology 177 (19), 5719-5722 (1995)),
- the pfkB gene coding for 6-phosphofructokinase II (Gene 28 (3), 337-342 (1984)),
- the malE gene coding for the periplasmatic binding protein of maltose transport (Journal of Biological Chemistry 259 (16), 10606-10613 (1984)),
- the rseA gene of the rseABC operon coding for a membrane protein with anti-sigmaE activity (Molecular Microbiology 24 (2), 355-371 (1997)),
- the rseC gene of the rseABC operon coding for a global regulator of the sigmaE factor (Molecular Microbiology 24 (2), 355-371 (1997)),
- the sodA gene coding for superoxide dismutase (Journal of Bacteriology 155 (3), 1078-1087 (1983)),
- the sucA gene of the sucABCD operon coding for the decarboxylase subunit of 2-ketoglutarate dehydrogenase (European Journal of Biochemistry 141 (2), 351-359 (1984)),
- the sucB gene of the sucABCD operon coding for the dihydrolipoyl transsuccinase E2 subunit of 2-ketoglutarate dehydrogenase (European Journal of Biochemistry 141 (2), 361-374 (1984)),
- the sucC gene of the sucABCD operon coding for the β subunit of succinyl-CoA synthetase (Biochemistry 24 (22), 6245-6252 (1985)), and
- the sucD gene of the sucABCD operon coding for the α subunit of succinyl-CoA synthetase (Biochemistry 24 (22), 6245-6252 (1985))
can be simultaneously enhanced and, in particular, overexpressed.

Furthermore, for the production of L-threonine, it can be advantageous not only to enhance the pepB gene but also to attenuate and, in particular, switch off one or more genes selected from the group comprising:
- the tdh gene coding for threonine dehydrogenase (Journal of Bacteriology 169, 4716-4721 (1987)),
- the mdh gene coding for malate dehydrogenase (E.C. 1.1.1.37) (Archives in Microbiology 149, 36-42 (1987)),
- the gene product of the open reading frame (orf) yjfA (Accession Number AAC77180 of the National Center for Biotechnology Information (NCBI, Bethesda, MD, USA)),
- the gene product of the open reading frame (orf) ytfP (Accession Number AAC77179 of the National Center for Biotechnology Information (NCBI, Bethesda, MD, USA)),
- the pckA gene coding for the enzyme phosphoenolpyruvate carboxykinase (Journal of Bacteriology 172, 7151-7156 (1990)),
- the poxB gene coding for pyruvate oxidase (Nucleic Acids Research 14 (13), 5449-5460 (1986)),
- the aceA gene coding for the enzyme isocitrate lyase (Journal of Bacteriology 170, 4528-4536 (1988)),
- the dgsA gene coding for the DgsA regulator of the phosphotransferase system (Bioscience, Biotechnology and Biochemistry 59, 256-251 [sic] 1995)), which is also known as the mlc gene,
- the fruR gene coding for the fructose repressor (Molecular and General Genetics 226, 332-336 (1991)), which is also known as the cra gene,
- the rpoS gene coding for the sigma³⁸ factor (WO 01/05939), which is also known as the katF gene,
- the aspA gene coding for aspartate ammonium lyase (aspartase) (Nucleic Acids Research 13 (6), 2063-2074 (1985)), and
- the aceB gene coding for malate synthase A (Nucleic Acids Research 16 (19), 9342 (1988)),
or reduce the expression.

In this context the term "attenuation" describes the decrease or switching-off of the intracellular activity, in a microorganism, of one or more enzymes (proteins) coded for by the appropriate DNA, for example by using a weak promoter or using a gene or allele which codes for an appropriate enzyme with a low activity or inactivates the appropriate enzyme (protein) or gene, and optionally combining these measures.

The attenuation measures generally reduce the activity or concentration of the appropriate protein to 0 to 75%, 0 to 50%, 0 to 25%, 0 to 10% or 0 to 5% of the activity or concentration of the wild-type protein or of the activity or concentration of the protein in the starting microorganism.

Furthermore, for the production of L-threonine, it can be advantageous not only to enhance the pepB gene but also to switch off unwanted secondary reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms prepared according to the invention can be cultivated by the batch process, the fed batch process or the repeated fed batch process. A summary of known cultivation methods is provided in the textbook by Chmiel (Bioprozesstechnik 1. Einführung in die Bioverfahrenstechnik (Bioprocess Technology 1. Introduction to Bioengineering) (Gustav Fischer Verlag, Stuttgart, 1991)) or in the textbook by Storhas (Bioreaktoren und periphere Einrichtungen (Bioreactors and Peripheral Equipment) (Vieweg Verlag, Brunswick/Wiesbaden, 1994)).

The culture medium to be used must appropriately meet the demands of the particular strains. Descriptions of culture media for various microorganisms can be found in "Manual of Methods for General Bacteriology" of the American Society for Bacteriology (Washington DC, USA, 1981).

Carbon sources which can be used are sugars and carbohydrates, e.g. glucose, sucrose, lactose, fructose, maltose, molasses, starch and optionally cellulose, oils and fats, e.g. soya oil, sunflower oil, groundnut oil and coconut fat, fatty acids, e.g. palmitic acid, stearic acid and linoleic acid, alcohols, e.g. glycerol and ethanol, and organic acids, e.g. acetic acid.. These substances can be used individually or as a mixture.

Nitrogen sources which can be used are organic nitrogen-containing compounds such as peptones, yeast extract, meat extract, malt extract, corn steep liquor, soya flour and urea, or inorganic compounds such as ammonium sulfate, ammonium chloride, ammonium phosphate, ammonium carbonate and ammonium nitrate. The nitrogen sources can be used individually or as a mixture.

Phosphorus sources which can be used are phosphoric acid, potassium dihydrogenphosphate or dipotassium hydrogenphosphate or the corresponding sodium salts. The culture medium must also contain metal salts, e..g.. magnesium sulfate or iron sulfate, which are necessary for growth. Finally, essential growth-promoting substances such as amino acids and vitamins can be used in addition to the substances mentioned above. Suitable precursors can also be added to the culture medium. Said feed materials can be added to the culture all at once or fed in appropriately during cultivation.

The fermentation is generally carried out at a pH of 5.5 to 9.0, especially of 6.0 to 8.0. The pH of the culture is controlled by the appropriate use of basic compounds such as sodium hydroxide, potassium hydroxide, ammonia or aqueous ammonia, or acid compounds such as phosphoric acid or sulfuric acid.. Foaming can be controlled using antifoams such as fatty acid polyglycol esters. The stability of plasmids can be maintained by adding suitable selectively acting substances, e.g. antibiotics, to the medium. Aerobic conditions are maintained by introducing oxygen or oxygen-containing gaseous mixtures, e.g. air, into the culture. The temperature of the culture is normally 25°C to 45°C and preferably 30°C to 40°C. The culture is continued until the formation of L-amino acids or L-threonine has reached a maximum. This objective is normally achieved within 10 hours to 160 hours.

L-Amino acids can be analyzed by means of anion exchange chromatography followed by ninhydrin derivatization, as described by Spackman et al. (Analytical Chemistry 30, 1190-1206 (1958)), or by reversed phase HPLC, as described by Lindroth et al. (Analytical Chemistry 51, 1167-1174 (1979)).

The process according to the invention is used for the preparation of L-threonine by fermentation.

The incubation temperature in the preparation of strains and transformants is 37°C.

### Example 1

### Construction of expression plasmid pTrc99ApepB

The pepB gene from E. coli K12 is amplified using the polymerase chain reaction (PCR) and synthetic oligonucleotides. The nucleotide sequence of the pepB gene in E. coli K12 MG1655 (Accession Number AE000339, Blattner et al.. (Science 277, 1453-1474 (1997))) is used as the starting material to synthesize PCR primers (MWG Biotech, Ebersberg, Germany). The 5' ends of the primers are extended with recognition sequences for restriction enzymes and with two to four additional bases. The recognition sequences for BamHI and SalI, which are underlined in the nucleotide sequence shown below, are chosen for the pepB5' and pepB3' primers respectively:
pepB5': 5'-CGCGGATCC-AACTGGCGGCCCTTT-'3' (SEQ ID No. 1)
pepB3': 5'-ACGCGTCGAC-CTGATGCGCTACGCT-3' (SEQ ID No. 2)

The chromosomal E. coli K12 MG1655 DNA used for the PCR is isolated with "Qiagen Genomic-tips 100/G" (QIAGEN, Hilden, Germany) in accordance with the manufacturer's instructions. An approx. 1440 bp DNA fragment can be amplified with the specific primers under standard PCR conditions (Innis et al. (1990) PCR Protocols. A Guide to The L-threonine-producing E. coli strain MG442 is described in patent US-A-4,278,765 and is deposited in the Russian National Collection for Industrial Microorganisms (VKPM, Moscow, Russia) as CMIM B-1628.

The strain MG442 is transformed with expression plasmid pTrc99ApepB, described in Example 1, and with vector pTrc99A and plasmid-carrying cells are selected on LB agar supplemented with 50 µg/ml of ampicillin. This procedure yields the strains MG442/pTrc99ApepB and MG442/pTrc99A. Chosen individual colonies are then multiplied further on minimum medium of the following composition: 3.5 g/l of Na₂HPO₄·2H₂O, 1.5 g/l of KH₂PO₄, 1 g/l of NH₄Cl, 0.1 g/l of MgSO₄·7H₂O, 2 g/l of glucose, 20 g/l of agar, 50 mg/l of ampicillin. The formation of L-threonine is verified in 10 ml batch cultures contained in 100 ml conical flasks. This is done by inoculating 10 ml of preculture medium of the following composition: 2 g/l of yeast extract, 10 g/l of (NH₄)₂SO₄, 1 g/l of KH₂PO₄, 0.5 g/l of MgSO₄·7H₂O, 15 g/l of CaCO₃, 20 g/l of glucose, 50 mg/l of ampicillin, and incubating for 16 hours at 37°C and 180 rpm on an ESR incubator from Kühner AG (Birsfelden, Switzerland). 250 µl of each of these precultures are transferred to 10 ml of production medium (25 g/l of (NH₄)₂SO₄, 2 g/l of KH₂PO₄, 1 g/l of MgSO₄·7H₂O, 0.03 g/l of FeSO₄·7H₂O, 0.018 g/l of MnSO₄·1H₂O, 30 g/l of CaCO₃, 20 g/l of glucose, 50 mg/l of ampicillin) and incubated for 48 hours at 37°C. The formation of L-threonine by the original strain MG442 is verified in the same way except that no ampicillin is added to the medium. After incubation the optical density (OD) of the culture suspension is determined using an LP2W photometer from Dr. Lange (Düsseldorf, Germany) at a measurement wavelength of 660 nm.

The concentration of L-threonine formed is then determined in the sterile-filtered culture supernatant using an amino acid analyzer from Eppendorf-BioTronik (Hamburg, Germany) by means of ion exchange chromatography and postcolumn reaction with ninhydrin detection.

Table 1 shows the result of the experiment.

**Table 1**

| Strain | OD (660 nm) | L-threonine g/l |
|---|---|---|
| MG442 | 5.6 | 1.4 |
| MG442/pTrc99A | 3.8 | 1.3 |
| MG442/pTrc99ApepB | 4.9 | 2.2 |

### Brief Description of the Figure:

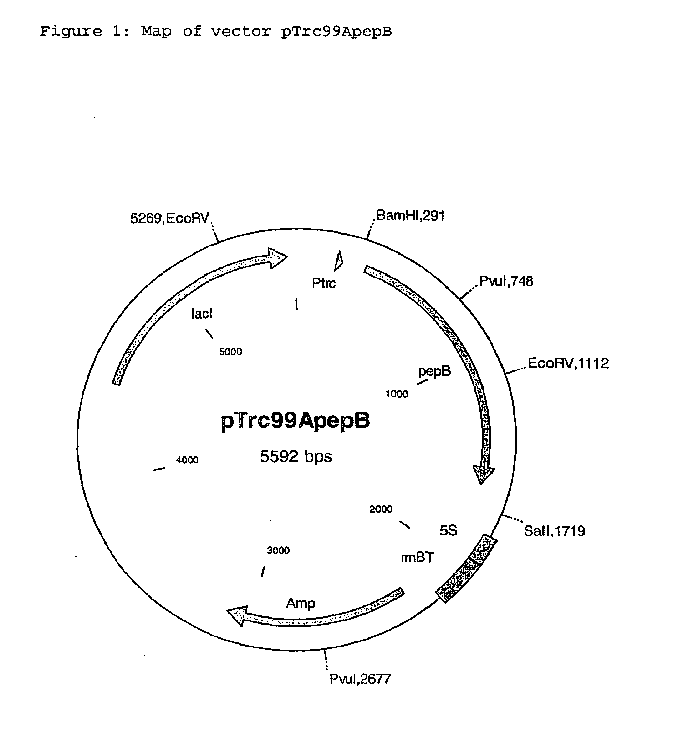

### Figure 1: Map of vector pTrc99ApepB

The indicated lengths are to be understood as approximate. The abbreviations and symbols used are defined as follows:

| | |
|---|---|
| • Amp: | ampicillin resistance gene |
| • lacI: | gene for the repressor protein of the trc promoter |
| • Ptrc: | trc promoter region, IPTG-inducible |
| • pepB: | coding region of the pepB gene |
| • 5S: | 5S rRNA region |
| • rmBT: | rRNA terminator region |

The abbreviations for the restriction enzymes are defined as follows:

| | |
|---|---|
| • BamHI: | restriction endonuclease from *Bacillus amyloliquefaciens* H |
| • EcoRV: | restriction endonuclease from *Escherichia coli* B946 |
| • SalI: | restriction endonuclease from *Streptomyces albus G* |
| • PvuI: | restriction endonuclease from *Proteus vulgaris* |

## Claims

1. A process for the preparation of steps are. L-threonine, wherein following steps are carried out:
a) fermentation of microorganisms of the family Enterobacteriaceae which produce L-threonine in which the pepB gene, or nucleotide sequence coding for the pepB gene product, is (are) enhanced
b) enrichment of L-threonine in the medium or in the cells of the microorganisms, and
c) isolation of L-threonine where all or part of the biomass, optionally remain in prepared L-threonine.

2. The process according to claim 1, wherein microorganisms are used in which other genes of the biosynthetic pathway of L-threonine are additionally enhanced.

3. The process according to claim 1, wherein microorganisms are used in which the metabolic pathways which reduce the formation of L-threonine are at least partially switched off.

4. The process according to claim 1, wherein the expression of the polynucleotide(s) coding for the pepB gene product is increased.

5. The process according to claim 1, wherein the regulatory and/or catalytic properties of the polypeptide (protein) coded for by the polynucleotide pepB are improved or enhanced.

6. The process according to claim 1, wherein microorganisms of the family Enterobacteriaceae in which additionally one or more genes selected from the group comprising:
6.1 the thrABC operon coding for aspartate kinase, homoserine dehydrogenase, homoserine kinase and threonine synthase,
6.2 the pyc gene coding for pyruvate carboxylase,
6.3 the pps gene coding for phosphoenolpyruvate synthase,
6.4 the ppc gene coding for phosphoenolpyruvate carboxylase,
6.5 the pntA and pntB genes coding for transhydrogenase,
6.6 the rhtB gene for homoserine resistance,
6.7 the mqo gene coding for malate:quinone oxidoreductase,
6.8 the rhtC gene for threonine resistance,
6.9 the thrE gene coding for threonine export protein,
6.10 the gdhA gene coding for glutamate dehydrogenase,
6.11 the hns gene coding for DNA binding protein HLP-II,
6.12 the pgm gene coding for phosphoglucomutase,
6.13 the fba gene coding for fructose biphosphate aldolase,
6.14 the ptsH gene coding for phosphohistidine protein hexose phosphotransferase,
6.15 the ptsI gene coding for enzyme I of the phosphotransferase system,
6.16 the crr gene coding for the glucose-specific IIA component,
6.17 the ptsG gene coding for the glucose-specific IIBC component,
6.18 the lrp gene coding for the regulator of the leucine regulon,
6.19 the csrA gene coding for the global regulator Csr,
6.20 the fadR gene coding for the regulator of the fad regulon,
6.21 the iclR gene coding for the regulator of the central intermediary metabolism,
6.22 the mopH gene coding for the 10 kd chaperone,
6.23 the ahpC gene coding for the small subunit of alkyl hydroperoxide reductase,
6.24 the ahpF gene coding for the large subunit of alkyl hydroperoxide reductase,
6.25 the cysK gene coding for cysteine synthase A,
6.26 the cysB gene coding for the regulator of the cys regulon,
6.27 the cysJ gene coding for the flavoprotein of NADPH sulfite reductase,
6.28 the cysI gene coding for the hemoprotein of NADPH sulfite reductase,
6.29 the cysH gene coding for adenylyl sulfate reductase,
6.30 the phoB gene coding for the PhoB positive regulator of the pho regulon,
6.31 the phoR gene coding for the sensor protein of the pho regulon,
6.32 the phoE gene coding for protein E of the outer cell membrane,
6.33 the pykF gene coding for fructose-stimulated pyruvate kinase I,
6.34 the pfkB gene coding for 6-phosphofructokinase II,
6.35 the malE gene coding for the periplasmatic binding protein of maltose transport,
6.36 the rseA gene coding for a membrane protein with anti-sigmaE activity,
6.37 the rseC gene coding for a global regulator of the sigmaE factor,
6.38 the sodA gene coding for superoxide dismutase,
6.39 the sucA gene coding for the decarboxylase subunit of 2-ketoglutarate dehydrogenase,
6.40 the sucB gene coding for the dihydrolipoyl transsuccinase E2 subunit of 2-ketoglutarate dehydrogenase,
6.41 the sucC gene coding for the β subunit of succinyl-CoA synthetase, and
6.42 the sucD gene coding for the a subunit of succinyl-CoA synthetase
is (are) simultaneously enhanced are fermented for the preparation of L-threonine.

7. The process according to claim 1, wherein microorganisms of the family Enterobacteriaceae in which additionally one or more genes selected from the group comprising:
7.1 the tdh gene coding for threonine dehydrogenase,
7.2 the mdh gene coding for malate dehydrogenase,
7.3 the gene product of the open reading frame (orf) yj fA,
7.4 the gene product of the open reading frame (orf) ytfP,
7.5 the pckA gene coding for phosphoenolpyruvate carboxykinase,
7.6 the poxB gene coding for pyruvate oxidase,
7.7 the aceA gene coding for isocitrate lyase,
7.8 the dgsA gene coding for the DgsA regulator of the phosphotransferase system,
7.9 the fruR gene coding for the fructose repressor,
7.10 the rpoS gene coding for the sigma³⁸ factor,
7.11 the aspA gene coding for aspartate ammonium lyase (aspartase), and
7.12 the aceB gene coding for malate synthase A
is (are) simultaneously attenuated or switched off are fermented for the preparation of L-threonine.

8. Microorganisms of the family Enterobacteriaceae, which produce L-threonine in which the pepB gene, or nucleotide sequences coding for the pepB gene product is (are) enhanced. and one or more genes selected from the group comprising:
8.1 the thrABC operon coding for aspartate kinase, homoserine dehydrogenase, homoserine kinase and threonine synthase,
8.2 the pyc gene coding for pyruvate carboxylase,
8.3 the pps gene coding for phosphoenolpyruvate synthase,
8.4 the ppc gene coding for phosphoenolpyruvate carboxylase,
8.5 the pntA and pntB genes coding for transhydrogenase,
8.6 the rhtB gene for homoserine resistance,
8.7 the mqo gene coding for malate:quinone oxidoreductase,
8.8 the rhtC gene for threonine resistance,
8.9 the thrE gene coding for threonine export protein,
8.10 the gdhA gene coding for glutamate dehydrogenase,
8.14 the ptsH gene coding for phosphohistidine protein hexose phosphotransferase,
8.15 the ptsI gene coding for enzyme I of the phosphotransferase system,
8.16 the crr gene coding for the glucose-specific IIA component,
8.17 the ptsG gene coding for the glucose-specific IIBC component,
8.18 the lrp gene coding for the regulator of the leucine regulon,
8.19 the csrA gene coding for the global regulator Csr,
8.20 the fadR gene coding for the regulator of the fad regulon,
8.21 the iclR gene coding for the regulator of the central intermediary metabolism,
8.22 the mopB gene coding for the 10 kd chaperone,
8.23 the ahpC gene coding for the small subunit of alkyl hydroperoxide reductase,
8. 24 the ahpF gene coding for the large subunit of alkyl hydroperoxide reductase,
8.26 the cysB gene coding for the regulator of the cys regulon,
8.27 the cysJ gene coding for the flavoprotein of NADPH sulfite reductase,
8.28 the cysI gene coding for the hemoprotein of NADPH sulfite reductase,
8.29 the cysH gene coding for adenylyl sulfate reductase,
8.30 the phoB gene coding for the PhoB positive regulator of the pho regulon,
8.31 the phoR gene coding for the sensor protein of the pho regulon,
8.32 the phoE gene coding for protein E of the outer cell membrane,
8.33 the pykF gene coding for fructose-stimulated pyruvate kinase I,
8.34 the pfkB gene coding for 6-phosphofructokinase II,
8.35 the malE gene coding for the periplasmatic binding protein of maltose transport,
8.36 the rseA gene coding for a membrane protein with anti-sigmaE activity,
8.37 the rseC gene coding for a global regulator of the sigmaE factor,
8.38 the sodA gene coding for superoxide dismutase,
8.39 the sucA gene coding for the decarboxylase subunit of 2-ketoglutarate dehydrogenase,
8.40 the sucB gene coding for the dihydrolipoyl transsuccinase E2 subunit of 2-ketoglutarate dehydrogenase,
8.41 the sucC gene coding for the β subunit of succinyl-CoA synthetase, and
8.42 the sucD gene coding for the α subunit of succinyl-CoA synthetase
are simultaneously enhanced

9. Microorganisms according to claim 8, whereby they belong to the genus Escherichia.

## Patentansprüche

1. Verfahren für die Herstellung von L-Threonin, wobei folgende Schritte durchgeführt werden.
a) Fermentation von Mikroorganismen der Familie Enterobacteriaceae, welche L-Threonin produzieren, in welchen das pepB-Gen oder Nukleotidsequenzen, die das pepB-Genprodukt codieren, verstärkt ist (sind);
b) Anreicherung von L-Threonin in dem Medium oder in den Zellen der Mikroorganismen; und
c) Isolierung von L-Threonin, wobei die ganze oder ein Teil der Biomasse gegebenenfalls in dem hergestellten L-Threonin zurückbleibt.

2. Verfahren gemäß Anspruch 1, bei dem Mikroorganismen verwendet werden, in welchen andere Gene des Biosynthesewegs von L-Threonin zusätzlich verstärkt sind.

3. Verfahren gemäß Anspruch 1, bei dem Mikroorganismen verwendet werden, in denen die Stoffwechselwege, welche die Bildung von L-Threonin reduzieren, zumindest teilweise abgeschaltet sind.

4. Verfahren gemäß Anspruch 1, bei dem die Expression des Polynukleotids bzw. der Polynukleotide, die das pepB-Genprodukt codieren, erhöht ist.

5. Verfahren gemäß Anspruch 1, bei dem die regulatorischen und/oder katalytischen Eigenschaften des Polypeptids (Proteins), das von dem Polynukleotid pepB codiert wird, verbessert oder erhöht sind.

6. Verfahren gemäß Anspruch 1, bei dem Mikroorganismen der Familie Enterobacteriaceae, in denen zusätzlich ein oder mehrere Gene, die aus der Gruppe gewählt sind, welche Folgendes umfasst:
6.1 das thrABC-Operon, welches Aspartatkinase, Homoserindehydrogenase, Homoserinkinase und Threoninsynthase codiert,
6.2 das pyc-Gen, welches Pyruvatcarboxylase codiert,
6.3. das pps-Gen, welches Phosphoenolpyruvatsynthase codiert,
6.4 das ppc-Gen, welches Phosphoenolpyruvatcarboxylase codiert,
6.5 die pntA- und pntB-Gene, welche Transhydrogenase codieren,
6.6 das rhtB-Gen für Homoserin-Resistenz,
6.7 das mqo-Gen, welches Malat:Chinonoxidoreduktase codiert,
6.8 das rhtC-Gen für Threonin-Resistenz,
6.9 das thrE-Gen, welches Threoninexportprotein codiert,
6.10 das gdhA-Gen, welches Glutamatdehydrogenase codiert,
6.11 das hns-Gen, welches DNA-Bindungsprotein HLP-II codiert,
6.12 das pgm-Gen, welches Phosphoglucomutase codiert,
6.13 das fba-Gen, welches Fructosebiphosphataldolase codiert,
6.14 das ptsH-Gen, welches Phosphohistidinproteinhexosephosphotransferase codiert,
6.15 das ptsI-Gen, welches Enzym I des Phosphotransferasesystems codiert,
6.16 das crr-Gen, welches die Glucose-spezifische IIA-Komponente codiert,
6.17 das ptsG-Gen, welches die Glucose-spezifische IIBC-Komponente codiert,
6.18 das lrp-Gen, welches den Regulator des Leucinregulons codiert,
6.19 das csrA-Gen, welches den globalen Regulator Csr codiert,
6.20 das fadR-Gen, welches den Regulator des fad-Regulons codiert,
6.21 das ic1R-Gen, welches den Regulator des zentralen intermediären Metabolismus codiert,
6.22 das mopB-Gen, welches das 10-kd-Chaperon codiert,
6.23 das ahpC-Gen, welches die kleine Untereinheit von Alkylhydroperoxidreduktase codiert,
6.24 das ahpF-Gen, welches die große Untereinheit von Alkylhydroperoxidreduktase codiert,
6.25 das cysK-Gen, welches Cysteinsynthase A codiert,
6.26 das cysB-Gen, welches den Regulator des cys-Regulons codiert,
6.27 das cysJ-Gen, welches das Flavoprotein von NADPH-Sulfitreduktase codiert,
6.28 das cysI-Gen, welches das Hämoprotein von NADPH-Sulfitreduktase codiert,
6.29 das cysH-Gen, welches Adenylylsulfatreduktase codiert,
6.30 das phoB-Gen, welches den PhoB-positiven Regulator des pho-Regulons codiert,
6.31 das phoR-Gen, welches das Sensorprotein des pho-Regulons codiert,
6.32 das phoE-Gen, welches Protein E der äußeren Zellmembran codiert,
6.33 das pykF-Gen, welches Fructose-stimulierte Pyruvatkinase I codiert,
6.34 das pfkB-Gen, welches 6-Phosphofructokinase II codiert,
6.35 das malE-Gen, welches das periplasmatische Bindungsprotein des Maltosetransports codiert, 6.36 das rseA-Gen, welches ein Membranprotein mit anti-sigmaE-Aktivität codiert,
6.37 das rseC-Gen, welches einen globalen Regulator des sigmaE-Faktors codiert,
6.38 das sodA-Gen, welches Superoxiddismutase codiert,
6.39 das sucA-Gen, welches die Decarboxylase-Unterheit von 2-Ketoglutaratdehydrogenase codiert,
6.40 das sucB-Gen, welches die Dihydrolipoyltranssuccinase-E2-Unterheit von 2-Ketoglutaratdehydrogenase codiert,
6.41 das sucC-Gen, welches die β-Untereinheit von Succinyl-CoA-synthetase codiert, und
6.42 das sucD-Gen, welches die α-Untereinheit von Succinyl-CoA-synthetase codiert,
gleichzeitig verstärkt ist (sind), für die Herstellung von L-Threonin fermentiert werden.

7. Verfahren gemäß Anspruch 1, bei dem Mikroorganismen der Familie Enterobacteriaceae, in denen zusätzlich ein oder mehrere Gene, die aus der Gruppe gewählt sind, welche Folgendes umfasst:
7.1 das tdh-Gen, welches Threonindehydrogenase codiert,
7.2 das mdh-Gen, welches Malatdehydrogenase codiert,
7.3 das Genprodukt des offenen Leserahmens (orf) yjfA,
7.4 das Genprodukt des offenen Leserahmens (orf) ytfP,
7.5 das pckA-Gen, welches Phosphoenolpyruvatcarboxykinase codiert,
7.6 das poxB-Gen, welches Pyruvatoxidase codiert,
7.7 das aceA-Gen, welches Isocitratlyase codiert,
7.8 das dgsA-Gen, welches den DgsA-Regulator des Phosphotransferasesystems codiert,
7.9 das fruR-Gen, welches den Fructoserepressor codiert,
7.10 das rpoS-Gen, welches den sigma³⁸-Faktor codiert,
7.11 das aspA-Gen, welches Aspartatammoniumlyase (Aspartase) codiert, und
7.12 das aceB-Gen, welches Malatsynthase A codiert,
gleichzeitig abgeschwächt ist (sind) oder abgeschaltet ist (sind), für die Herstellung von L-Threonin fermentiert werden.

8. Mikroorganismen der Familie Enterobacteriaceae, welche L-Threonin produzieren, in welchen das pepB-Gen oder Nukleotidsequenzen, die das pepB-Genprodukt codieren, verstärkt ist (sind), und ein oder mehrere Gene, die aus der Gruppe gewählt sind, welche Folgendes umfasst:
8.1 das thrABC-Operon, welches Aspartatkinase, Homoserindehydrogenase, Homoserinkinase und Threoninsynthase codiert,
8.2 das pyc-Gen, welches Pyruvatcarboxylase codiert,
8.3. das pps-Gen, welches Phosphoenolpyruvatsynthase codiert,
8.4 das ppc-Gen, welches Phosphoenolpyruvatcarboxylase codiert,
8.5 die pntA- und pntB-Gene, welche Transhydrogenase codieren,
8.8 das rhtB-Gen für Homoserin-Resistenz,
8.7 das mqo-Gen, welches Malat:Chinonoxidoreduktase codiert,
8.8 das rhtC-Gen für Threonin-Resistenz,
8.9 das thrE-Gen, welches Threoninexportprotein codiert,
8.10 das gdhA-Gen, welches Glutamatdehydrogenase codiert,
8.14 das ptsH-Gen, welches Phosphohistidinproteinhexosephosphotransferase codiert,
8.15 das ptsI-Gen, welches Enzym I des Phosphotransferasesystems codiert,
8.18 das crr-Gen, welches die Glucose-spezifische IIA-Komponente codiert,
8.17 das ptsG-Gen, welches die Glucose-spezifische IIBC-Komponente codiert,
8.18 das lrp-Gen, welches den Regulator des Leucinregulons codiert,
8.19 das csrA-Gen, welches den globalen Regulator Csr codiert,
8.20 das fadR-Gen, welches den Regulator des fad-Regulons codiert,
8.21 das ic1R-Gen, welches den Regulator des zentralen intermediären Metabolismus codiert,
8.22 das mopB-Gen, welches das 10-kd-Chaperon codiert,
8.23 das ahpC-Gen, welches die kleine Untereinheit von Alkylhydroperoxidreduktase codiert,
8.24 das ahpF-Gen, welches die große Untereinheit von Alkylhydroperoxidreduktase codiert,
8.26 das cysB-Gen, welches den Regulator des cys-Regulons codiert,
8.27 das cysJ-Gen, welches das Flavoprotein von NADPH-Sulfitreduktase codiert,
8.28 das cysI-Gen, welches das Hämoprotein von NADPH-Sulfitreduktase codiert,
8.29 das cysH-Gen, welches Adenylylsulfatreduktase codiert,
8.30 das phoB-Gen, welches den PhoB-positiven Regulator des pho-Regulons codiert,
8.31 das phoR-Gen, welches das Sensorprotein des pho-Regulons codiert,
8.32 das phoE-Gen, welches Protein E der äußeren Zellmembran codiert,
8.33 das pykF-Gen, welches Fructose-stimulierte Pyruvatkinase I codiert,
8.34 das pfkB-Gen, welches 6-Phosphofructokinase II codiert,
8.35 das malE-Gen, welches das periplasmatische Bindungsprotein des Maltosetransports codiert,
8.36 das rseA-Gen, welches ein Membranprotein mit anti-sigmaE-Aktivität codiert,
8.37 das rseC-Gen, welches einen globalen Regulator des sigmaE-Faktors codiert,
8.38 das sodA-Gen, welches Superoxiddismutase codiert,
8.39 das sucA-Gen, welches die Decarboxylase-Unterheit von 2-Ketoglutaratdehydrogenase codiert,
8.40 das sucB-Gen, welches die Dihydrolipoyltranssuccinase-E2-Unterheit von 2-Ketoglutaratdehydrogenase codiert,
8.41 das sucC-Gen, welches die β-Untereinheit von Succinyl-CoA-synthetase codiert, und
8.42 das sucD-Gen, welches die α-Untereinheit von Succinyl-CoA-synthetase codiert,
gleichzeitig verstärkt ist bzw. sind.

9. Mikroorganismen gemäß Anspruch 8, wobei sie zum Genus Escherichia gehören.

## Revendications

1. Procédé pour la préparation de L-thréonine, dans lequel les étapes suivantes sont conduites :
a) fermentation de microorganismes de la famille des Enterobacteriacae produisant de la L-thréonine, dans lesquels le gène pepB, ou des séquences nucléotidiques codant pour le produit du gène pepB est (sont) amplifié(es),
b) enrichissement de la L-thréonine dans le milieu ou dans les cellules des micro-organismes, et
c) isolement de la L-thréonine, où toute ou une partie de la biomasse, reste éventuellement dans la L-thréonine préparée.

2. Procédé selon la revendication 1, **caractérisé en ce que** des microorganismes sont utilisés, dans lesquels d'autres gènes de la voie de biosynthèse de la L-thréonine sont également amplifiés.

3. Procédé selon la revendication 1, **caractérisé en ce que** des microorganismes sont utilisés dans lesquels les voies métaboliques qui réduisent la formation de L-thréonine souhaité sont au moins partiellement inactivées.

4. Procédé selon la revendication 1, **caractérisé en ce que** l'expression du (des) polynucléotide(s) codant pour le produit du gène pepB est augmentée.

5. Procédé selon la revendication 1, **caractérisé en ce que** les propriétés régulatrices et/ou catalytiques du polypeptide (protéine) codé par le polynucléotide pepB sont améliorées ou augmentées.

6. Procédé selon la revendication 1, **caractérisé en ce que** des microorganismes de la famille des Enterobacteriacae dans lesquels un ou plusieurs gènes choisis dans le groupe comprenant :
6.1 l'opéron thrABC codant pour l'aspartate kinase, l'homosérine déshydrogénase, l'homosérine kinase et la thréonine synthase,
6.2 le gène pyc codant pour la pyruvate carboxylase,
6.3 le gène pps codant pour la phosphoénolpyruvate synthase,
6.4 le gène ppc codant pour la phosphoénolpyruvate carboxylase,
6.5 les gènes pntA et pntB codant pour la transhydrogénase,
6.6 le gène rhtB pour la résistance à l'homosérine,
6.7 le gène mqo codant pour la malate:quinone oxydoréductase,
6.8 le gène rhtC pour la résistance à la thréonine,
6.9 le gène thrE codant pour la protéine d'export de thréonine,
6.10 le gène gdhA codant pour la glutamate déshydrogénase,
6.11 le gène hns codant pour la protéine de liaison d'ADN HLP-II,
6.12 le gène pgm codant pour la phosphoglucomutase,
6.13 le gène fba codant pour la fructose biphosphate aldolase,
6.14 le gène ptsH codant pour la phosphohistidine protéine hexose phosphotransférase,
6.15 le gène ptsI codant pour l'enzyme I du système phosphotransférase,
6.16 le gène crr codant pour le composant IIA spécifique du glucose,
6.17 le gène ptsG codant pour le composant IIBC spécifique du glucose,
6.18 le gène lrp codant pour le régulateur du régulon leucine,
6.19 le gène csrA codant pour le régulateur global Csr,
6.20 le gène fadR codant pour le régulateur du régulon fad,
6.21 le gène iclR codant pour le régulateur du métabolisme intermédiaire central,
6.22 le gène mopB codant pour le chaperon de 10 kd,
6.23 le gène ahpC codant pour la petite sous-unité de l'alkyl-hydroperoxyde réductase,
6.24 le gène ahpF codant pour la grande sous-unité de l'alkyl-hydroperoxyde réductase,
6.25 le gène cysK codant pour la cystéine synthase A,
6.26 le gène cysB codant pour le régulateur du régulon cys,
6.27 le gène cysJ codant pour le flavoprotéine de NADPH sulfite réductase,
6.28 le gène cysI codant pour l'hémoprotéine de NADPH sulfite réductase,
6.29 le gène cysH codant pour l'adénylyl-sulfate réductase,
6.30 le gène phoB codant pour le régulateur positif PhoB du régulon pho,
6.31 le gène phoR codant pour la protéine de détection du régulon pho,
6.32 le gène phoE codant pour la protéine E de la membrane cellulaire externe,
6.33 le gène pykF codant pour la pyruvate kinase I stimulée par le fructose,
6.34 le gène pfkB codant pour la 6-phosphofructokinase II,
6.35 le gène malE codant pour la protéine de liaison périplasmique de transport du maltose,
6.36 le gène rseA codant pour une protéine membranaire avec une activité anti-sigmaE, 6.37 le gène rseC codant pour un régulateur global du facteur sigmaE,
6.38 le gène sodA codant pour la superoxyde dismutase,
6.39 le gène sucA codant pour la sous-unité décarboxylase de 2-cétoglutarate déshydrogénase,
6.40 le gène sucB codant pour la sous-unité dihydrolipyle trans-succinase E2 de 2-cétoglutarate déshydrogénase,
6.41 le gène sucC codant pour la sous-unité β de succinyl-CoA synthase, et
6.42 le gène sucD codant pour la sous-unité α de succinyl-CoA synthase,
est (sont) simultanément amplifiés, sont fermentés pour la préparation de L-thréonine.

7. Procédé selon la revendication 1, **caractérisé en ce que** des micro-organismes de la famille des Enterobacteriacae dans lesquels un ou plusieurs des gènes choisis dans le groupe comprenant :
7.1 le gène tdh codant pour la thréonine déshydrogénase,
7.2 le gène mdh codant pour la malate déshydrogénase,
7.3 le produit génique du cadre ouvert de lecture (ORF) yjfA,
7.4 le produit génique du cadre ouvert de lecture (ORF) ytfP,
7.5 le gène pckA codant pour la phosphoénolpyruvate carboxylase,
7.6 le gène poxB codant pour la pyruvate oxydase,
7.7 le gène aceA codant pour l'isocitrate lyase,
7.8 le gène dgsA codant pour le régulateur DgsA du système phosphotransférase,
7.9 le gène fruR codant pour le répresseur de fructose,
7.10 le gène rpoS codant le facteur sigma³⁸,
7.11 le gène aspA codant pour l'aspartate ammonium lyase (aspartate), et
7.12 le gène aceB codant pour la malate synthase A est (sont) simultanément atténué (s) ou inactivé(s), sont fermentés pour la préparation de L-thréonine.

8. Micro-organismes de la famille des Enterobacteriacae produisant de la L-thréonine, dans lesquels le gène pepB, ou des séquences nucléotidiques codant pour le produit du gène pepB est (sont) amplifié(es) et un ou plusieurs gènes choisis dans le groupe comprenant :
8.1 l'opéron thrABC codant pour l'aspartate kinase, l'homosérine déshydrogénase, l'homosérine kinase et la thréonine synthase,
8.2 le gène pyc codant pour la pyruvate carboxylase,
8.3 le gène pps codant pour la phosphoénolpyruvate synthase,
8.4 le gène ppc codant pour la phosphoénolpyruvate carboxylase,
8.5 les gènes pntA et pntB codant pour la transhydrogénase,
8.6 le gène rhtB pour la résistance à l'homosérine,
8.7 le gène mqo codant pour la malate:quinone oxydoréductase,
8.8 le gène rhtC pour la résistance à la thréonine,
8.9 le gène thrE codant pour la protéine d'export de thréonine,
8.10 le gène gdhA codant pour la glutamate déshydrogénase,
8.14 le gène ptsH codant pour la phosphohistidine protéine hexose phosphotransférase,
8.15 le gène ptsI codant pour l'enzyme I du système phosphotransférase,
8.16 le gène crr codant pour le composant IIA spécifique du glucose,
8.17 le gène ptsG codant pour le composant IIBC spécifique du glucose,
8.18 le gène lrp codant pour le régulateur du régulon leucine,
8.19 le gène csrA codant pour le régulateur global Csr,
8.20 le gène fadR codant pour le régulateur du régulon fad,
8.21 le gène iclR codant pour le régulateur du métabolisme intermédiaire central,
8.22 le gène mopB codant pour le chaperon de 10 kd,
8.23 le gène ahpC codant pour la petite sous-unité de l'alkyl-hydroperoxyde réductase,
8.24 le gène ahpF codant pour la grande sous-unité de l'alkyl-hydroperoxyde réductase,
8.26 le gène cysB codant pour le régulateur du régulon cys,
8.27 le gène cysJ codant pour le flavoprotéine de NADPH sulfite réductase,
8.28 le gène cysI codant pour l'hémoprotéine de NADPH sulfite réductase,
8.29 le gène cysH codant pour l'adénylyl-sulfate réductase,
8.30 le gène phoB codant pour le régulateur positif PhoB du régulon pho,
8.31 le gène phoR codant pour la protéine de détection du régulon pho,
8.32 le gène phoE codant pour la protéine E de la membrane cellulaire externe,
8.33 le gène pykF codant pour la pyruvate kinase I stimulée par le fructose,
8.34 le gène pfkB codant pour la 6-phosphofructokinase II,
8.35 le gène malE codant pour la protéine de liaison périplasmique de transport du maltose,
8.36 le gène rseA codant pour une protéine membranaire avec une activité anti-sigmaE, 8.37 le gène rseC codant pour un régulateur global du facteur sigmaE,
8.38 le gène sodA codant pour la superoxyde dismutase,
8.39 le gène sucA codant pour la sous-unité décarboxylase de 2-cétoglutarate déshydrogénase,
8.40 le gène sucB codant pour la sous-unité dihydrolipyle trans-succinase E2 de 2-cétoglutarate déshydrogénase,
8.41 le gène sucC codant pour la sous-unité β de succinyl-CoA synthase, et
8.42 le gène sucD codant pour la sous-unité α de succinyl-CoA synthase,
sont simultanément amplifiés.

9. Micro-organismes selon la revendication 8, **caractérisé en ce qu'**ils appartiennent au genre Escherichia.


### SEQUENCE LISTING

<110> Degussa AG
<120> Process for the preparation of L-amino acids using strains of the family Enterobacteriaceae
<130> 020117 BT
<160> 2
<170> PatentIn version 3.1
<210> 1
   <211> 24
   <212> DNA
   <213> Artificial sequence
<220>
   <221> Primer
   <222> (1)..(24)
   <223> pepB5'
<400> 1
   cgcggatcca actggcggcc cttt 24
<210> 2
   <211> 25
   <212> DNA
   <213> Artificial sequence
<220>
   <221> Primer
   <222> (1)..(25)
   <223> pepB3'
<400> 2
   acgcgtcgac ctgatgcgct acgct 25